# EUROPÄISCHE PATENTSCHRIFT

(11) **EP 0 719 758 B1**
(45) Veröffentlichungstag und Bekanntmachung des Hinweises auf die Patenterteilung: **30.12.1998**
(21) Anmeldenummer: 95120589.7
(22) Anmeldetag: 27.12.1995
(51) Int. Cl.: C07C 67/343, C07C 69/738, C07C 69/734, C07C 69/618, C07C 205/56, C07C 41/30, C07C 43/243, C07C 45/68, C07C 49/794, C07C 49/217, C07C 49/255, C07C 205/06, C07C 47/548, C07C 49/796

(54) **Verfahren zur Herstellung aromatischer Olefine**
Process for the production of aromatic olefines
Procédé de préparation d'oléfines aromatiques

(30) Priorität: 29.12.1994 DE 4447068
(43) Veröffentlichungstag der Anmeldung: 03.07.1996
(73) Patentinhaber: Celanese GmbH, 60439 Frankfurt (DE)
(72) Erfinder: Herrmann, Wolfgang A., Prof. Dr., D-85354 Freising (DE); Fischer, Jakob, D-85414 Kirchdorf (DE); Elison, Martina, D-80798 München (DE); Köcher, Christian, D-80805 München (DE)

(56) Entgegenhaltungen:
- EP-A- 0 103 544
- WO-A-93/01173

## Beschreibung

Die vorliegende Erfindung betrifft ein Verfahren zur Herstellung aromatischer Olefine mit neuartigen Palladium-Katalysatoren, die als besonderes Merkmal Carben-Liganden aus der Heterocyclen-Reihe aufweisen.

Aromatische Olefine, insbesondere Zimtsäurederivate, Styrole, Stilbene, haben technische Bedeutung als Feinchemikalien, als Ausgangsstoffe für Polymere, als UV-Absorber und als Vorprodukte für pharmazeutische Wirkstoffe. Eine häufig angewandte Methode zu ihrer Synthese ist die Heck-Reaktion, d.h. die Umsetzung von Iod- oder Bromaromaten und in Ausnahmefällen Chloraromaten mit Olefinen in Gegenwart von Palladiumkatalysatoren. Übersichten, die diese Methodik ausführlich beschreiben, findet man beispielsweise in R.F. Heck, Acc. Chem. Res. 1979, 12, 146; R.F. Heck, Org. React. 1982, 27, 345; R.F. Heck, Palladium Reagents in Synthesis, Academic Press, London 1985. In der wissenschaftlichen und in der Patentliteratur werden als Katalysatoren für diese Reaktion Phosphin-Komplexe von Palladium(0) und Palladium(II) beschrieben. Auch Palladium-Kolloide sind katalytisch aktiv, doch ist ihre Leistungsfähigkeit durch die geringere thermische Beständigkeit stark eingeschränkt. Die Heck-Reaktion erfordert nämlich Temperaturen von 60 bis 140°C und höher. Daher sind nur solche Katalysatoren insbesondere für einen technischen Einsatz geeignet, die derartige thermische Belastungen auch über lange Zeiträume unzersetzt überstehen. Dies trifft in besonderer Weise auf die für technische Anwendungen bedeutsame Aktivierung von Chloraromaten in der Heck-Reaktion zu. Chloraromaten sind nämlich vielseitig verfügbare kostengünstige Ausgangsmaterialien. Nachteilig ist jedoch, daß Kohlenstoff-Chlor-Bindungen im Vergleich zu Kohlenstoff-Brom- und Kohlenstoff-Iod-Bindungen erheblich beständiger und deshalb weniger reaktiv sind.

Es besteht deshalb seit langem die Aufgabe, ausreichend aktive und selektive Palladium-Katalysatoren, die hohe thermische Beständigkeit auch bei Langzeit-Temperaturbelastung aufweisen, für die Heck-Reaktion von Halogenaromaten, insbesondere Chloraromaten, zu entwickeln.

Diese Aufgabe wird gelöst durch ein Verfahren zur Herstellung von mono-, di- oder polyfunktionellen aromatischen Olefinen durch Umsetzung von Halogenaromaten mit Olefinen. Es ist dadurch gekennzeichnet, daß man die Umsetzung bei Temperaturen von 20 bis 220°C in Gegenwart von Verbindungen als Katalysatoren durchführt, die der allgemeinen Formel

[LₐPd_{b}X_{c}]ⁿAₙ (I)

entsprechen, in der X an Palladium als Zentralatom gebundene ein- oder mehrzähnige, geladene oder ungeladene Liganden bedeutet und L ebenfalls an das Zentralatom als Liganden gebundene Monocarbene der allgemeinen Formeln 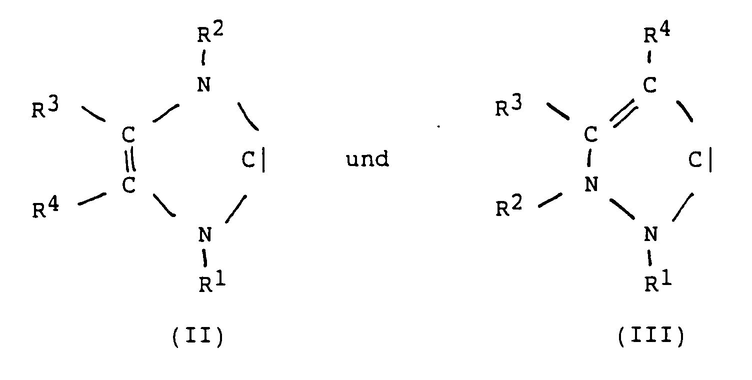 oder Dicarbene der allgemeinen Formeln 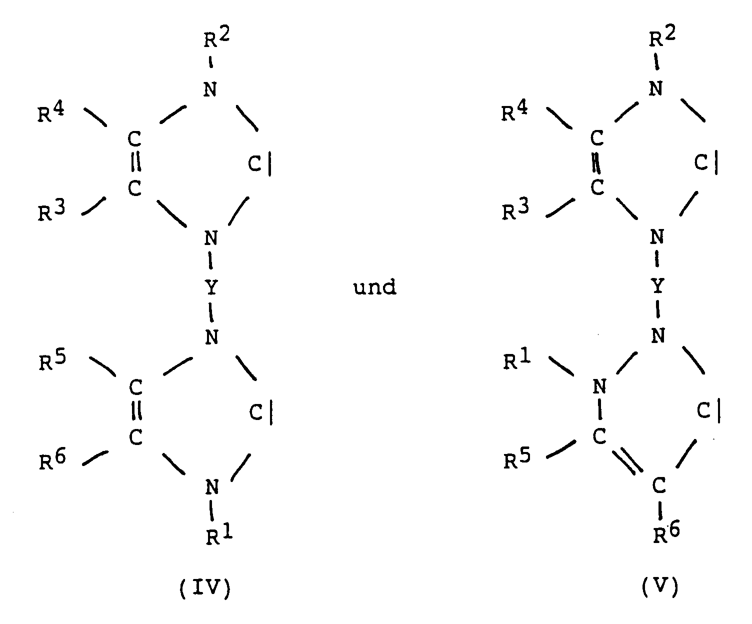 sind, wobei R¹, R², R³, R⁴, R⁵ und R⁶ gleiche oder verschiedene geradkettige oder verzweigte, gegebenenfalls sulfonierte Alkylreste mit 1 bis 7 Kohlenstoffatomen, gegebenenfalls sulfonierte aliphatische mono- oder polycyclische Reste mit 5 bis 18 Kohlenstoffatomen, gegebenenfalls sulfonierte Alkenylreste mit 2 bis 5 Kohlenstoffatomen, gegebenenfalls sulfonierte Arylreste mit 6 bis 14 Kohlenstoffatomen oder gegebenenfalls sulfonierte Arylalkylreste mit 7 bis 19 Kohlenstoffatomen bedeuten, R³, R⁴, R⁵ und R⁶ auch für Wasserstoff stehen, R³ und R⁴ gemeinsam sowie R⁵ und R⁶ gemeinsam jeweils auch gleiche oder verschiedene anellierte und gegebenenfalls sulfonierte Reste mit 3 bis 7 Kohlenstoffatome bedeuten, R¹, R², R⁴ oder R⁶ mit Liganden X einen Cyclus bilden können, Y ein gesättigter oder ungesättigter, geradkettiger oder verzweigter Alkylidenrest mit 1 bis 4 Kohlenstoffatomen oder ein Dialkylsilylen- oder ein Tetraalkyldisylilenrest ist, A für ein einfach geladenes Anion oder das chemische Äquivalent eines mehrfach geladenen Anions steht, b eine ganze Zahl von 1 bis 3 darstellt, a einer ganzen Zahl von 1 bis 4 · b und c = 0 oder einer ganzen Zahl von 1 bis 4 · b entsprechen und n = 0 oder eine ganze Zahl von 1 bis 6 ist.

Überraschenderweise haben sich Komplexverbindungen des Palladiums, die als Liganden Carbene oder Dicarbene enthalten, die sich vom Imidazol oder vom Pyrazol und deren Derivaten ableiten, als sehr aktive und selektiv wirkende Katalysatoren für Umsetzungen von Halogenaromaten mit Olefinen zu aromatischen Olefinen erwiesen. Von besonderer Bedeutung ist die strukturelle Vielfalt der als Liganden eingesetzten Carbene, die mit Palladium in seinen verschiedenen Oxidationsstufen Komplexverbindungen bilden und die Herstellung spezifisch wirksamer Katalysatoren ermöglichen. Diese Katalysatoren sind thermisch oft bis weit oberhalb 300°C stabil und beständig gegen die Einwirkung von Sauerstoff und anderen milden Oxidationsmitteln. Die Katalysatoren sind in der Regel auch wasserbeständig.

Als ein- oder mehrzähnige Liganden, die neben den Carbenen in den katalytisch wirksamen Komplexverbindungen enthalten sein können und in der allgemeinen Formel (I) durch X wiedergegeben werden, sind Wasserstoff oder das Wasserstoff-Ion, Halogene oder Halogen-Ionen, Pseudohalogenide, Carboxylat-Ionen, Sulfonat-Ionen, Alkylgruppen mit 1 bis 7 Kohlenstoffatomen Amidreste, Alkoholatreste, Acetylacetonatreste, Kohlenmonoxid, Stickstoffmonoxid, Nitrile, Isonitrile, Mono- oder Diolefine, Alkine und π-Aromatenreste. Sind mehrere dieser Liganden im Komplexmolekül enthalten, dann können sie gleich oder verschieden sein.

In den vom Imidazol und vom Pyrazol oder deren Derivaten abgeleiteten Mono- bzw. Dicarbenen entsprechend den Formeln (II), (III), (IV) und (V) stehen R¹ bis R⁶ insbesondere für die Reste Methyl, Isopropyl, tert.-Butyl, Benzyl, Triphenylmethyl, Phenyl, Tolyl, Xylyl und Mesityl. R³ und R⁴ stehen vorzugsweise für Wasserstoff und die Methylgruppe.

Die Reste R³ und R⁴ und die Reste R⁵ und R⁶ können zusammen mit zwei benachbarten Kohlenstoffatomen des Imidazolringes oder des Pyrazolrings ein Ringsystem bilden. R³ und R⁴ bzw. R⁵ und R⁶ stehen dann bevorzugt für die Gruppierungen (CH)₄, sie führt zur Ausbildung eines anellierten aromatischen Sechsrings, (CH₂)₄ und (CH₂)₅.

Die durch Y bezeichneten Brückenglieder der Dicarbene gemäß den Formeln (IV) und (V) sind vorzugsweise die Methylen-, Dimethylmethylen-, Diphenylmethylen-, 1,3-Phenylen- und die Ethylidengruppe. Unter den Silizium enthaltenden Brückengliedern werden die Dimethylsilylen- und die Tetramethyldisilylen-Gruppe bevorzugt.

a ist vorzugsweise 1 oder 2, b vorzugsweise 1; c vorzugsweise 0 bis 2; n steht insbesondere für die Zahlen 0 bis 2.

A vertritt bevorzugt Halogenid-, Pseudohalogenid-, Tetraphenylborat-, Tetrafluoroborat-, Hexafluorophosphat- und Carboxylat-Ionen, unter den zuletzt genannten insbesondere das Acetat-Ion, ferner Metallkomplex-Anionen wie z.B. Tetracarbonylcobaltat, Hexafluoroferrat(III), Tetrachloroferrat, Tetrachloroaluminat oder Tetrachloropalladat (II).

Beispiele für Verbindungen, die mit Erfolg als Katalysatoren eingesetzt werden, sind
Bis(1,3-dimethylimidazolin-2-yliden)palladium(II)-dichlorid, -dibromid und -diiodid
Bis(1-methyl-3-tritylimidazolin-2-yliden)palladium(O)
Bis(1,3-dimethylimidazolin-2-yliden)palladium(II)-bisacetylacetonat und -tetrachloroplatinat
Bis(1,3-diphenylimidazolin-2-yliden)palladium(II)-acetat, -trifluoroacetat und -trifluormethylsulfonat
Bis(acetonitril)-bis(1,3-diisopropylimidazolin-2-yliden)palladium(II)-bis(tetrafluoroborat) und -bis(tetraphenyloborat)
Bis(1,3-diphenylimidazolin-2-yliden)palladium(II)-bisacetat, -bis(trifluoroacetat) und -bis(trifluoromethylsulfonat)
Bis(1,3-dimethylbenzimidazolin-2-yliden)palladium(II)-dibromid und -diiodid
Bis(1,3-dimethylpyrazolin-yliden)palladium(II)-dibromid und
   -diiodid
(1,1'-Methylen-3,3'-dimethylimidazolin-2-yliden)palladium(II)-diiodid

Die als Einsatzstoffe verwendeten aromatischen Halogenverbindungen entsprechen der allgemeinen Formel

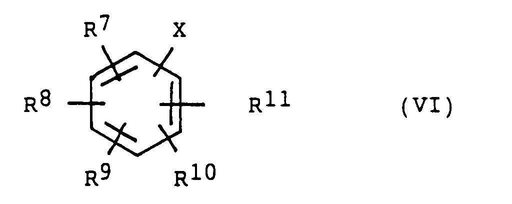

In dieser Formel bedeuten X Fluor, Chlor, Brom, Jod, R⁷ bis R¹¹ unabhängig voneinander Wasserstoff, Alkylreste mit 1 bis 8 Kohlenstoffatomen, Alkoxyreste mit 1 bis 8 Kohlenstoffatomen, Acyloxyreste mit 1 bis 8 Kohlenstoffatomen, -C₆H₅, OC₆H₅, Fluor, Chlor, Brom, Jod, -OH, -NO₂, -S(O)O₂CF₃, -CN, -COOH, -CHO, -SO₃H, -SO₂ (C₁- bis C₈-alkyl), -SO(C₁- bis C₈-alkyl), -NH₂, -NH(C₁- bis C₈-alkyl), -N(C₁- bis C₈-alkyl)₂, -C(hal)₃ (hal = Halogen), -NHCO(C₁- bis C₄-alkyl), -COO(C₁- bis C₈-alkyl), -CONH₂, -CO(C₁- bis C₈-alkyl), -NHCOOH, -NCOO(C₁- bis C₄-alkyl), -COC₆H₅, -COOC₆H₅, -PO(C₆H₅)₂ und -PO(C₁- bis C₄-alkyl).

Insbesondere sind R⁷ bis R¹¹ unabhängig voneinander Wasserstoff, Alkylreste mit 1 bis 8 Kohlenstoffatomen, Alkoxyreste mit 1 bis 8 Kohlenstoffatomen, -C₆H₅, Fluor, Chlor, -NO₂,
-CN, -COOH, -COO(C₁- bis C₈-alkyl), -CONH₂, -CO(C₁- bis C₈-alkyl), -COC₆H₅ und -PO(C₆H₅)₂. Einer der Reste R⁷ bis R¹¹ kann auch der Gruppierung 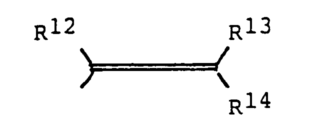 entsprechen, in der R¹² für Wasserstoff, Alkylreste mit 1 bis 8 Kohlenstoffatomen, Alkoxyreste mit 1 bis 8 Kohlenstoffatomen, den Phenylrest oder Fluor steht und R¹³ und R¹⁴ unabhängig voneinander Wasserstoff, -CN, -COOH, -COO(C₁- bis C₈-alkyl), -CONH₂, -CONH(C₁- bis C₄-alkyl), -CON(C₁- bis C₄-alkyl)₂, Fluor, -COOC₆H₅, (C₁- bis C₈-alkyl)C₆H₄, -PO(C₆H₅)₂, -PO[(C₁- bis C₄-alkyl)]₂, -COC₆H₅, -CO(C₁- bis C₄-alkyl), Alkoxyreste mit 1 bis 4 Kohlenstoffatomen, -NH(C₁- bis C₄-alkyl), -PO₃H, -SO₃H, -SO₃(C₁- bis C₄-alkyl), -SO₂(C₁- bis C₄-alkyl) oder -OC₆H₅ sind.

Reaktionspartner der vorstehend beschriebenen aromatischen Halogenverbindungen sind Olefine der allgemeinen Formel

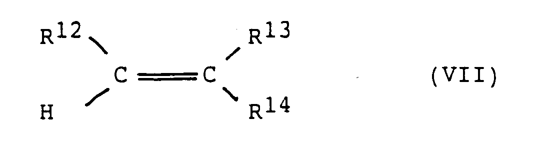

Die Reste R¹², R¹³ und R¹⁴ haben die oben wiedergegebene Bedeutung. In der Formel (VI) steht X bevorzugt für Chlor und Brom. Ferner sind R⁷, R⁸, R¹⁰ und R¹¹ insbesondere gleich und bedeuten Wasserstoff. R⁹ ist insbesondere der Methylrest, der Methoxyrest, -NO₂ und -C(O)H. In Formel (VII) bedeutet R¹² vorzugsweise Wasserstoff, einen Alkylrest mit 1 bis 8 Kohlenstoffatomen und insbesondere Wasserstoff. R¹³ und R¹⁴ sind unabhängig voneinander bevorzugt Wasserstoff, -CN, -COOH, -COO(C₁- bis C₈-alkyl), -COOC₆H₅, -COC₆H₅, -CO(C₁- bis C₄-alkyl) und besonders bevorzugt -CN, -COOH, -COO(C₁- bis C₈-alkyl) und -COOC₆H₅. Schließlich sind R¹² und R¹⁴ gleich und stehen für Wasserstoff.

Die Reaktion der Ausgangsstoffe erfolgt bei Temperaturen von 20 bis 220°C. In vielen Fällen hat es sich bewährt, bei 60 bis 180°C, vorzugsweise 100 bis 160°C zu arbeiten.

Im allgemeinen setzt man die Reaktanten in einem inerten, organischen Lösungsmittel um. Gut geeignet sind dipolar aprotische Lösungsmittel, wie Dialkylsulfoxide, N,N-Dialkylamide aliphatischer Carbonsäuren oder alkylierte Lactame. Bevorzugt verwendet werden Dimethylsulfoxid, Dimethylacetamid, Dimethylformamid, N-Methylpyrrolidon, Amine und Polyether.

Im Verlauf der Reaktion wird Halogenwasserstoff abgespalten, den man zweckmäßig durch Zusatz einer Base abfängt. Als Base geeignet sind primäre, sekundäre oder tertiäre Amine, z.B. Alkylamine, Dialkylamine, Trialkylamine, die alicyclisch oder offenkettig sein können, sowie Alkali- oder Erdalkalisalze der Kohlensäure oder aliphatischer oder aromatischer Carbonsäuren, z.B. die Carbonate, Hydrogencarbonate oder Acetate der Metalle Lithium, Natrium, Kalium, Calzium und Magnesium.

Aufgrund ihrer hohen Aktivität und Stabilität reichen bereits kleine Mengen der neuen Katalysatoren zur Durchführung der Umsetzung aus. Das Verfahren ist daher sehr wirtschaftlich und ökologisch vorteilhaft, weil Abfallprodukte vermieden werden und energieaufwendige Aufarbeitungsverfahren entbehrlich sind. Üblicherweise setzt man die Katalysatoren, bezogen auf die aromatische Halogenverbindung, in Mengen zwischen 10⁻⁴ bis 5 mol-%, vorzugsweise 10⁻² bis 0,5 mol-% ein.

Die Katalysatoren werden in der Regel vor der eigentlichen Reaktion isoliert synthetisiert, sie können jedoch auch im Reaktionsgemisch aus gängigen Palladiumverbindungen erzeugt werden, ohne daß dadurch die anfängliche katalytische Aktivität gemindert wird. Bei längerer Reaktionsführung erweisen sich die im Reaktionsgemisch erzeugten Katalysatoren, in denen das Palladium/Ligand-Verhältnis 1 : 1 bis 1 : 2 beträgt, jedoch als weniger stabil als die gesondert hergestellten Katalysatoren und führen häufiger zur Abscheidung von Palladium. Als Palladiumvorstufen eignen sich insbesondere die Palladium(II)-halogenide, Palladium(II)-acetat, Palladium(II)-acetylacetonat, Nitrilkomplexe der Palladium(II)-halogenide, Bis(dibenzylidenaceton)palladium(O) und Bis(1,5-cyclooctadien)palladium(O).

Die Herstellung der Katalysatoren in einem eigenen Reaktionsschritt erfolgt aus einfachen Verbindungen, d.h. Palladiumsalzen oder aus Komplexverbindungen des Palladiums durch Ligandenaustausch durch Anlagerungs-, Eliminierungs- und/oder Substitutionsreaktionen. Die Carbene werden entsprechend ihrer Beständigkeit entweder in freier Form als Lösung eingesetzt oder, häufiger, im Reaktionsgemisch aus Verbindungen hergestellt, die unter den Reaktionsbedingungen in Carbene überführt werden können. Die wichtigste Erzeugungsmethode ist die Deprotonierung von Imidazolium- oder Pyrazoliumsalzen, gegebenenfalls durch Zusatz von Basen wie Metallalkoholate, Metallhydride, Halogenmetallate oder Metallamide.

Die Aktivität der Katalysatoren läßt sich durch Zusatz von Alkalisalzen, Erdalkalisalzen oder Salzen von Übergangsmetallen der 6. bis 8. Nebengruppe erhöhen. Insbesondere der Zusatz von Halogeniden und Pseudohalogeniden wie Cyanid, bewirkt bei der Umsetzung von Chloraromaten erhebliche Ausbeutesteigerung und erhöht die Lebensdauer des homogenen Katalysators. Das gleiche Ergebnis erzielt man auch bei Zusatz von Tri- und Tetraalkylammonium-Salzen sowie den entsprechenden Phosphonium- und Arsoniumsalzen.

In den nachfolgenden Beispielen wird das erfindungsgemäße Verfahren erläutert, nicht jedoch auf diese speziellen Ausführungsformen beschränkt.

### Beispiel 1

### Herstellung von cis-Diiodo-bis(1,3-dimethylimidazolin-2-yliden)palladium(II) (Katalysator 1)

0,200 g (0,89 mmol) Palladium(II)-acetat werden in 25 ml absolutem Tetrahydrofuran (THF) bei Raumtemperatur mit 2,1 Äquivalenten 1,3-Dimethylimidazoliumiodid (0,420 g, 1,87 mmol) versetzt. Nach 30 min Erhitzen unter Rückfluß klart die vormals dunkelbraune Lösung nach gelb auf. Das Lösungsmittel wird im Hochvakuum abgezogen und der Rückstand dreimal mit 20 ml absolutem Diethylether gewaschen. Nach Umkristallisieren aus Methylenchlorid/Hexan bei 25°C erhält man 0,37 g des Katalysators 1 als gelben kristallinen Feststoff (Ausbeute: 370 mg = 75 %). Zersetzung bei 299°C.

### Charakterisierung

| C₁₀H₁₆N₄I₂Pd (552,5) | | | | |
|---|---|---|---|---|
| Analyse | ber | C 21,73 | H 2,92 | N 10,14 |
| | gef | C 23,26 | H 3,45 | N 10,00 |
| (Kristallisiert mit 1/2 Mol CH₂Cl₂) | | | | |

¹H-NMR (400 MHz, CDCl₃, 20°C, ppm):
δ H = 3,92 (s, 12H; N-Methyl), 7,24 (s, 4H; Imidazol).

¹³C-NMR (100.53 MHz, CDCl₃, 20°C, ppm):
δ C = 168,18 (Carben-C), 122,32 (Imidazol), 38,22 (N-Methyl).

Die Arbeitsvorschrift kann ohne Ausbeutenverminderung auf den 10- bis 100-fachen Ansatz angewandt werden, was auch für die weiteren Beispiele der Katalysatorherstellung gilt.

### Beispiel 2

### Herstellung von cis-Diiodo(1,1'-methylen-3,3'-dimethylimidazolin-2,2'-diyliden)palladium(II) (Katalysator 2)

0,200 g (0,89 mmol) Palladium(II)-acetat werden in 10 ml absolutem Toluol bei 25°C mit 0,400 g (0,89 mmol) 1,1'-Methylen-3,3'-dimethylimidazolium-diiodid versetzt. Nach 2 h Erhitzen unter Rückfluß wird die von dunkelrot nach gelb aufgeklarte Lösung mit Hilfe einer Kanüle filtriert. Die erhaltene gelbe Lösung wird im Hochvakuum eingedampft. Der Rückstand wird dreimal mit 10 ml absolutem Diethylether und 20 ml THF gewaschen. Man erhält den Katalysator als gelben Feststoff (Ausbeute: 290 mg = 61 %).

### Charakterisierung

| C₉H₁₂N₄I₂Pd (536.4) | | | | | |
|---|---|---|---|---|---|
| Analyse | ber | C 20,15 | H 2,25 | N 10,44 | I 47,31 |
| | gef | C 22,53 | H 2,78 | N 11,42 | I 47,68 |
| (Kristallisiert mit 1/2 Mol THF) | | | | | |

¹H-NMR (400 MHz, CDCl₃, 20°C, ppm):
δ H = 3,92 (s, 6H; N-Methyl), 6,61 (s, 2H, CH₂), 7,41 und 7,43 (s, 4H; Imidazol).

¹³C-NMR (100,53 MHz, CDCl₃, 20°C, ppm)
δ C = 36,31 (N-Methyl), 53,60 (CH₂), 121,87 und 124,35 (Imidazol), 185,50 (Carben-C).

### Beispiel 3

### Herstellung des Katalysators Bis(1,3-dimethylimidazolin-2-yliden)-palladium(II)-diacetat (Katalysator 3)

500 mg (2,2 mmol) Palladium(II)-acetat werden in 80 ml Toluol mit 4,4 mmol 1,3-Dimethylimidazolin-2-yliden (erhalten durch vorangehende in situ-Bildung aus 1,3-Dimethylimidazolium-iodid mittels Kalium-tert.-butylat und Natriumhydrid) in Toluol/THF bei Raumtemperatur umgesetzt. Der resultierende gelbe Niederschlag wird nach dreimal Waschen mit Ether und Umkristallisieren aus Methylenchlorid/Hexan im Hochvakuum getrocknet.

### Beispiel 4

### Herstellung von in situ-Katalysatoren

a) 70 mg (0,3 mmol) Palladium(II)-acetat werden mit 120 mg (0,6 mmol) 1-Methyl-3-isopropylimidazolium-bromid in 10 ml Dimethylacetamid (DMAc) versetzt. Diese Lösung kann bei Raumtemperatur unter Argonatmosphäre einige Zeit unverändert aufbewahrt werden. Für die Anwendung als Katalysator gemäß Beispiel 5 (Tab. 1) wird ein aliquoter Teil der Lösung entnommen, wobei sich die Einsatzmenge auf das Palladium(II)-acetat bezieht. Der aktive Katalysator ("Katalysator 4a") entwickelt sich unter den Reaktionstemperaturen nach Beispiel 5 (Tab. 1).
b) 70 mg (0,3 mmol) Palladium(II)-acetat werden mit 110 mg (0,3 mmol) 1,2-Bis(3-methylimidazolium-bromid)ethylen in 10 ml DMAc umgesetzt. Diese Reaktionslösung wird analog zu Beispiel 4a) bei Bedarf in der Katalyse eingesetzt. Der aktive Katalysator ("Katalysator 4b") entwickelt sich unter den Reaktionstemperaturen nach Beispiel 5 (Tab. 1).
c) 70 mg (0,12 mmol) Bis(dibenzylidenaceton)palladium(O) werden mit 23 mg (0,24 mmol) 1,3-Dimethylimidazolin-2-yliden (wie in Beispiel 3 angegeben hergestellt) in Toluollösung 15 min bei 25°C umgesetzt. Die resultierende Lösung wird mit Sauerstoff begast. Der entstehende grüne Niederschlag ist der aktive Katalysator ("Katalysator 4c") nach Beispiel 5. Er wird mehrmals mit Toluol, Ether und n-Pentan gewaschen und in den erforderlichen Mengen (Tab. 1) der jeweiligen Katalyselösung zugesetzt.
d) Herstellung des Carben-Palladium(O)-Katalysators Dibenzylidenacetonbis(1,3-dimethylimidazolin-2-yliden)palladium(O)

Bei Raumtemperatur wird eine Lösung von 200 mg (dba)₂Pd (dba = Dibenzylidenaceton) (0.348 mmol) in 40 ml Toluol unter strengem Luft- und Feuchtigkeitsausschluß portionsweise mit einer Lösung von 1,3-Dimethylimidazolin-2-yiliden (0.7 mmol) in THF (wie in Beispiel 3 angegeben hergestellt) versetzt und 10 min gerührt. Es erfolgt augenblicklich ein Farbumschlag von violett nach grün-rot. Das Lösungsmittel wird im Hochvakuum abdestilliert und der Rückstand in 10 ml entgastem Dimethylacetamid gelöst. Die Lösung wird sofort als Katalysatorlösung verwendet, kann aber auch über 24 h bei Raumtemperatur gelagert werden. Sie enthält den aktiven Carben-Palladium(O)-Komplex.

Zur Charakterisierung des Carben-Palladium(O)-Komplexes wird der Rückstand der Hochvakuumdestillation mehrmals mit trockenem Diethylether (jeweils 10 ml) gewaschen, bis die Waschlösung nicht mehr durch das freigesetzte Dibenzylidenaceton gelb gefärbt ist. Da das Produkt geringfügig löslich in Diethylether ist, sollte die Waschlösung auf etwa -20°C vorgekühlt werden. Das Produkt wird im Hochvakuum acht Stunden getrocknet. Nach Umkristallisation durch Überschichten einer Toluol-Lösung mit n-Pentan verbleibt ein grüner Feststoff.

Ausbeute: 150 mg (81 %)

Struktur:

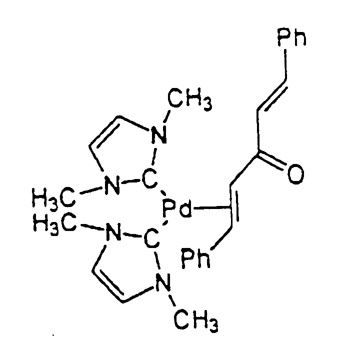

¹H-NMR (400 MHz, D8-Toluol, 20°C, ppm): δ = 7.05 (s, 4H, CH=CH), 3.84 (s, 12H, N-CH₃);
7.55 (breit, 4H), 7.35 (breit, 6H), 6.90 (breit, 4H); dba

IR (KBr, cm-1): 3161, 3121, 3023, 2923, 2846, 1636, 1471, 1401, 1229, 1085, 1028, 746, 689, 536.

### Beispiel 5

### Katalytische Herstellung von aromatischen Olefinen

Die Umsetzungen folgen dem folgenden Reaktionsschema:

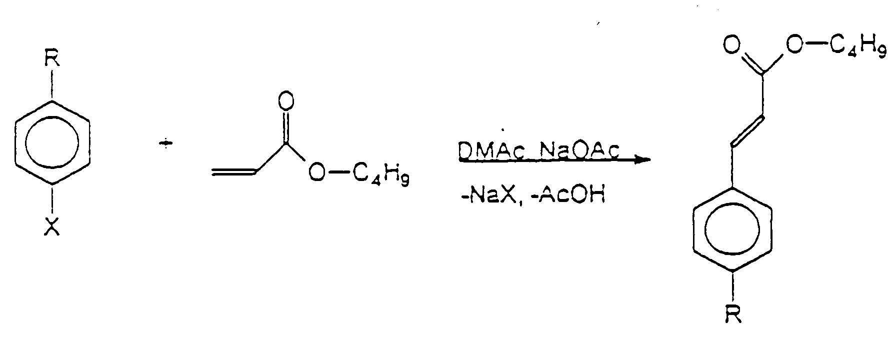

Die Reaktionsansätze werden in ausgeheizten Glasapparaturen mit angeschmolzenem Rückflußkühler in N₂- oder Ar-Atmosphäre durchgeführt. Der Reaktionsverlauf wird in regelmäßigen Zeitabständen mittels eines Gaschromatographen, der mit einem Flammenionisationsdetektor, einem Massenspektrometer und einem Infrarotspektrometer gekoppelt ist, analytisch verfolgt und quantifiziert.

In einem 100 ml Dreihalskolben mit Septum, Innenthermometer und Rückflußkühler werden 6 mmol Halogenaromat, 8 mmol wasserfreies Natriumacetat und 0,1 g Diethylenglykol-di-n-butylether (GC-Standard) in 10 ml N,N-Dimethylacetamid vorgelegt.

Nach mehrfachem Entgasen unter vermindertem Druck und anschließendem Spülen mit Stickstoff werden 10 mmol n-Butylacrylat über das Septum injiziert. Der Ansatz wird auf 120°C erwärmt. Bei Erreichen der Temperatur wird die Katalysatormischung bzw. Lösung des Pd-Carbenkomplexes über das Septum eingespritzt (sofern nicht anders in Tabelle 1 angegeben: 0,03 mmol Katalysator entsprechend 0,5 mol-% Katalysator bezüglich des Halogenaromats) und der Ansatz bis zur endgültigen Reaktionstemperatur von 140°C weiter erhitzt. Nach einer Reaktionszeit von, sofern nicht anders angegeben, 16 h werden die Reaktionsansätze durch Zugabe von Wasser und Extraktion der organischen Phase mit Methylenchlorid oder Diethylether aufgearbeitet. Nach Trocknung mit MgSO₄ und Entfernung der Lösungsmittel Methylenchlorid, Diethylether und Dimethylacetamid wird das erhaltene Rohprodukt durch Destillation oder Umkristallisation gereinigt.

Einzelheiten sind in Tabelle 1 zusammengefaßt; dort finden sich auch Hinweise auf Salzzusätze.

**Tabelle 1**

| (zu Beispiel 5) | | | |
|---|---|---|---|
| Nr. | Katalysator (mol-%) | Halogenaromat | Ausbeute, % |
| 1 | 1 | p-Brombenzaldehyd | >99 |
| 2 | 2 | p-Brombenzaldehyd | >99 |
| 3 | 4a | p-Brombenzaldehyd | >99 |
| 4 | 1 | p-Bromtoluol | 60 |
| 5 | 2 | p-Bromtoluol | 10 |
| 6 | 1 | p-Bromanisol | 35 |
| 7 | 2 | p-Bromanisol | 20 |
| 8 | 1 | 1.)p-Brombenzaldehyd | >99 nach 16 h |
| | | 2.)Brombenzol nach 32 h | 10 nach 48 h |
| 9 | 4c | p-Bromanisol | 55 nach 4 h |
| | | | 85 nach 72 h |
| 10 | 4c | p-Brom-N,N-dimethylanilin | 45 nach 4 h |
| | | | 70 nach 72 h |
| 11 | 4c | p-Chlornitrobenzol | 50 |
| 12 | 1 | p-Chlorbenzaldehyd | 7 |
| 13 | 1 | p-Chlorbenzaldehyd | >99 |
| 14 | 3 | p-Bromanisol | 48 |
| 15 | 4b | p-Chlorbenzaldehyd | 81 |
| 16 | 1 | p-Bromacetophenon | >99 |
| 17 | 1 | p-Bromacetophenon | >99 |
| 18 | 4d(0,1) | p-Bromacetophenon | >99 nach 1h |
| 19 | 4d(0,002) | p-Bromacetophenon | >99 nach 24h |
| 20 | 4d(0,0004) | p-Bromacetophenon | >99 nach 36h |
| 21 | 4d(0,1) | Brombenzol | 95 nach 8h |
| 22 | 4d(0,1) | p-Bromanisol | 80 nach 8h |
| 23 | 4d(0,2) | p-Chlornitrobenzol | 95 nach 24h |
| 24 | 4d(0,2) | p-Chlorbenzaldehyd | 80 nach 8h |

### Anmerkungen zur Tabelle 1

Nr. 8: Zu dem Reaktionsgemisch gemäß den obengenannten Reaktionsbedingungen werden erst nach 32 Stunden 6 mmol Brombenzol, 10 mmol n-Butylacrylat und 8 mmol Natriumacetat zugegeben. Obgleich der Katalysator dann bereits 32 Stunden thermisch belastet worden ist, ist die Katalysemischung bezüglich der obengenannten Reaktion noch aktiv.
Nr. 13 und 15: Bei Beibehaltung der obengenannten Reaktionsbedingungen werden vor Beginn der Reaktion zur Reaktionsmischung 7 mmol Tetra-n-butylammoniumbromid gegeben. Die Erhöhung der Ausbeute bei Umsatz von Chloraromaten gemäß obigem Reaktionsschema durch Zusatz von Tetra-n-butylammoniumbromid gilt allgemein.
Nr. 16: Nach 67 min wird das Reduktionsmittel Hydrazinhydrat zugegeben.
Nr. 17: Nach 60 min wird das Reduktionsmittel Natriumformiat zugegeben.
Nr. 24: Die Reaktion erfolgt unter Zusatz von 10 mol-% Tetra-n-butylammoniumbromid.

### Beispiel 6:

### Heck-Olefinierung durch Umsetzung von 2-Brom-6-methoxynaphthalin mit Ethylen im Autoklaven.

Die Umsetzungen folgen dem folgenden Reaktionsschema:

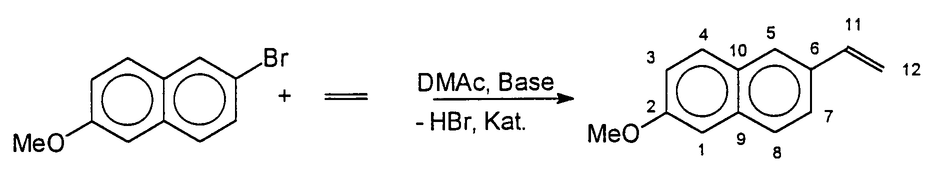

10,69 g (45 mmol) 2-Brom-6-methoxynaphthalin werden zusammen mit 50 mmol einer Base wie Natriumcarbonat, Natriumacetat, Natriumformiat oder Triethylamin, 0,9 g Ethylenglykoldi-n-butylether (GC-Standard)und 55 mg 2,4,6 Tri(tert.-butyl)phenol als Radikalfänger in einen Glaseinsatz eines Roth Laborautoklaven (250 ml, maximal 200 bar Gesamtdruck) eingewogen und mit 45 ml N,N-Dimethylacetamid als Lösungsmittel versetzt.

Für eine innige Durchmsichung sorgt ein Teflonrührstab, der durch einen unter dem Heizmantel des Autoklaven befindlichen Magnetrührer angetrieben wird. Nach Zugabe von 0,225 mmol des Carben-Palladium-(O)-Katalysators 4d wird der Autoklav verschlossen und bis zu einem Gesamtdruck von 50 bar mit Ethylen beschickt. Erst dann wird die Reaktionstemperatur auf 120°C gesteigert. Die Reaktion erfordert nur wenige Stunden, wird aber im allgemeinen, um vollständigen Umsatz zu gewährleisten, 24 Stunden fortgesetzt. Nach Abkühlen wird der Autoklav entspannt.

Die Aufarbeitung erfolgt analog zu Beispiel 5. Der Umsatz beträgt 80 %, die Ausbeute an 2-Methoxy-6-vinylnaphthalin ist > 78 %.

¹H-NMR (400 MHz, CDCl₃, 20°C, ppm)
5,15 (dd, 1H, 12-H, ³J = 10,9 Hz (cis)); 5,69 (dd, 1H, 12-H, ³J = 17,6 Hz (trans));
6,70 (dd, 1H, 11-H)

¹³C{¹H}-NMR(CDCl₃, 100,1 MHz, 20°C)
54,7 (OMe); 112,6 (C-12); 157,4 (C-2); 136,5 (C-11); 105,4; 118,5; 119,3; 123,3; 125,8; 126,6; 128,0; 129,1;

EI-MS: m/z (%) = 184 (M⁺, 100); 169 (M+-CH₃,23)

FT-IR (CH₂Cl₂ [cm⁻¹])
987(w), 902(w), δ (R-CH=CH2), 3064(w), 3010(w), 1603(s), 1484(m), ν(Aromat), 2846(w), 2948(w), ν(-OMe)

### Beispiel 7

Die Umsetzungen folgen dem folgenden Reaktionsschema:

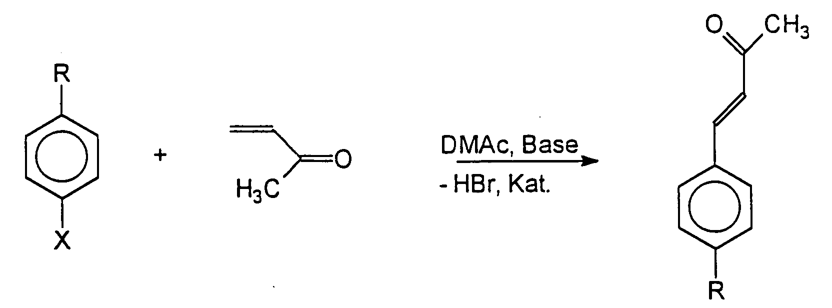

Die Durchführung der Umsetzung erfolgt wie in Beispiel 5 beschrieben, wobei folgende spezielle Reaktionsbedingungen gewählt werden:
25 mmol Halogenaromat
30 mmol Methylvinylketon
30 mmol Natriumacetat (analog können andere Basen wie Natriumcarbonat, Kaliumcarbonat usw. verwendet werden)
Katalysator 0,025 mmol entsprechend 0,1 Mol%
50 ml N,N-Dimethylacetamid als Lösungsmittel
0,5 g Ethylenglykoldi-n-butylether (GC-Standard)
Reaktionstemperatur 125°C.

Die erzielten Ausbeuten sind in Tabelle 2 aufgeführt.

**Tabelle 2**

| Nr. | Katalysator (Mol%) | Halogenaromat | Ausbeute, % |
|---|---|---|---|
| 1 | 4d (0,1) | p-Bromacetophenon | 99 nach 8 h |
| 2 | 4d (0,1) | Brombenzol | 99 nach 8h |
| 3 | 4d (0,1) | p-Bromanisol | 80 nach 8 h |

### Beispiel 8

Die Umsetzungen folgen dem folgenden Reaktionsschema:

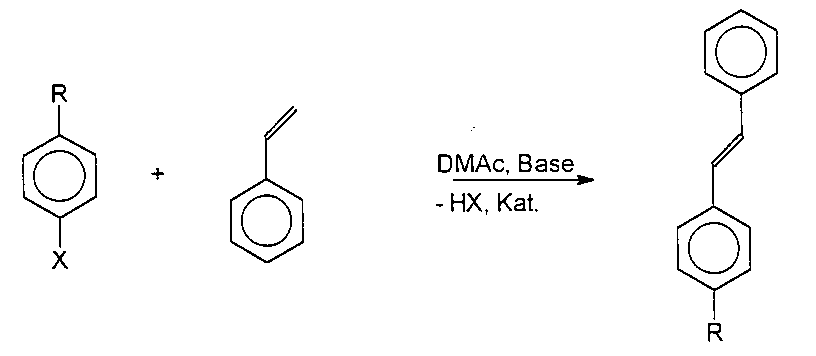

Die Durchführung der Reaktion erfolgt wie allgemein in Beispiel 5 beschrieben, wobei die folgenden speziellen Reaktionsbedingungen gewählt werden:
25 mmol Halogenaromat
30 mmol Styrol
30 mmol Natriumacetat (analog können andere Basen wie Trialkylamine, Natriumcarbonat, Kaliumcarbonat usw. verwendet werden)
Katalysator 0,025 mmol entsprechend 0,1 Mol% (sofern nicht anders angegeben)
0,5 g Ethylenglykoldi-n-butylether (GC-Standard )
55 mg 2,4,6-Tri(tert.-butyl)phenol (Radikalfänger)
50 ml N,N-Dimethylacetamid als Lösungsmittel
Reaktionstemperatur 130°C

**Tabelle 3**

| Nr. | Katalysator (Mol%) | Halogenaromat | Zusätze | Ausbeute % |
|---|---|---|---|---|
| 1 | 4d (0,1) | p-Bromacetophenon | keine | 99 nach 8 h |
| 2 | 4d (0,1) | Brombenzol | keine | 99 nach 8h |
| 3 | 4d (0,1) | p-Bromanisol | keine | 80 nach 8 h |
| 4 | 4d (0,5) | p-Nitrochlorbenzol | keine | 95 nach 8 h |
| 5 | 4d (0,5) | p-Chlorbenzaldehyd | [N(n-Bu)₄]⁺Br⁻ | 85 nach 1 h |
| 6 | 1 (0,5) | p-Chlorbenzaldehyd | [N(n-Bu)₄]⁺Br⁻ | 85 nach 1 h |
| 7 | 1 (0,5) | p-Chlorbenzaldehyd | [N(n-Bu)₄]⁺I⁻ | 85 nach 1 h |
| 8 | 1 (0,5) | p-Chlorbenzaldehyd | [N(n-Bu)₄]⁺Cl⁻ | 30 nach 1 h |
| 9 | 1 (0,5) | p-Chlorbenzaldehyd | [N(n-Bu)₄]⁺BF₄⁻ | 27 nach 1 h |
| 10 | 1 (0,5) | p-Chlorbenzaldehyd | NaI | 34 nach 1 h |

## Patentansprüche

1. Verfahren zur Herstellung von mono-, di- oder polyfunktionellen aromatischen Olefinen durch Umsetzung von Halogenaromaten mit Olefinen, dadurch gekennzeichnet, daß man die Umsetzung bei Temperaturen von 20 bis 220°C in Gegenwart von Verbindungen als Katalysatoren durchführt, die der allgemeinen Formel
[LₐPd_{b}X_{c}]ⁿAₙ (I)
entsprechen, in der X an das Palladium als Zentralatom gebundene ein- oder mehrzähnige, geladene oder ungeladene Liganden bedeutet und L ebenfalls an das Zentralatom als Liganden gebundene Monocarbene der allgemeinen Formeln 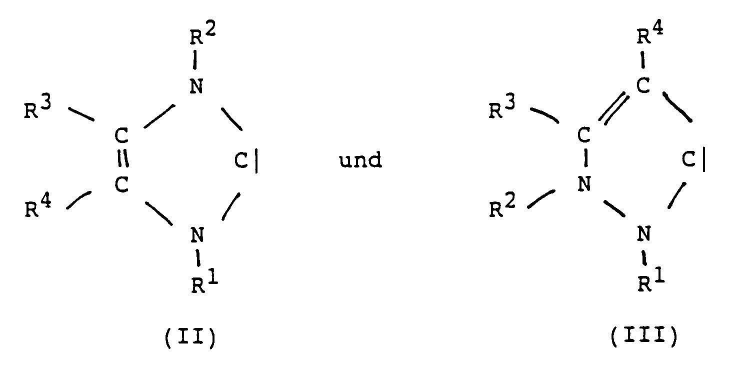 oder Dicarbene der allgemeinen Formeln 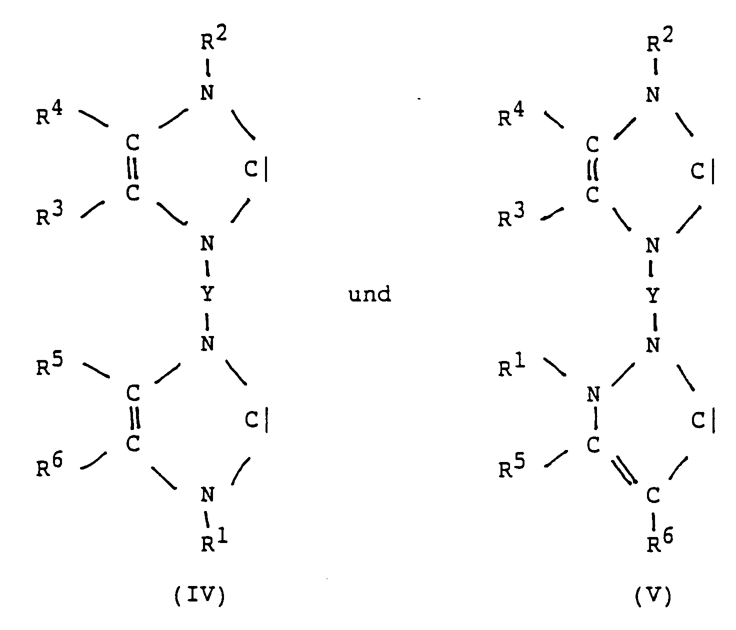 sind, wobei R¹, R², R³, R⁴, R⁵ und R⁶ gleiche oder verschiedene geradkettige oder verzweigte, gegebenenfalls sulfonierte Alkylreste mit 1 bis 7 Kohlenstoffatomen, gegebenenfalls sulfonierte aliphatische mono- oder polycyclische Reste mit 5 bis 18 Kohlenstoffatomen, gegebenenfalls sulfonierte Alkenylreste mit 2 bis 5 Kohlenstoffatomen, gegebenenfalls sulfonierte Arylreste mit 6 bis 14 Kohlenstoffatomen oder gegebenenfalls sulfonierte Arylalkylreste mit 7 bis 19 Kohlenstoffatomen bedeuten, R³, R⁴, R⁵ und R⁶ auch für Wasserstoff stehen, R³ und R⁴ gemeinsam sowie R⁵ und R⁶ gemeinsam jeweils auch gleiche oder verschiedene anellierte und gegebenenfalls sulfonierte Reste mit 3 bis 7 Kohlenstoffatomen bedeuten, R¹, R², R⁴ oder R⁶ mit Liganden X einen Cyclus bilden können, Y ein gesättigter oder ungesättigter, geradkettiger oder verzweigter Alkylidenrest mit 1 bis 4 Kohlenstoffatomen oder ein Dialkylsilylen- oder ein Tetraalkyldisilylenrest ist, A für ein einfach geladenes Anion oder das chemische Äquivalent eines mehrfach geladenen Anions steht, b eine ganze Zahl von 1 bis 3 darstellt, a einer ganzen Zahl von 1 bis 4 · b und c = 0 oder einer ganzen Zahl von 1 bis 4 · b entsprechen und n = 0 oder eine ganze Zahl von 1 bis 6 ist.

2. Verfahren nach Anspruch 1 dadurch gekennzeichnet, daß X in der allgemeinen Formel (I) für Wasserstoff, das Wasserstoff-Ion, Halogene, Halogen-Ionen, Pseudohalogenide, Carboxylat-Ionen, Sulfonat-Ionen, Alkylgruppen mit 1 bis 7 Kohlenstoffatomen, Amidreste, Alkoholatreste, Acetylacetonatreste, Kohlenmonoxid, Stickstoffmonoxid, Nitrile, Isonitrile, Mono- oder Diolefine, Alkine und π-Aromatenreste steht.

3. Verfahren nach Anspruch 1 oder 2 dadurch gekennzeichnet, daß in den allgemeinen Formeln (II), (III), (IV) und (V) R¹, R², R³, R⁴, R⁵, R⁶ für die Reste Methyl, Isopropyl, tert.-Butyl, Benzyl, Triphenylmethyl, Phenyl, Tolyl, Xylyl und Mesityl stehen.

4. Verfahren nach einem oder mehreren der Ansprüche 1 bis 3 dadurch gekennzeichnet, daß in den allgemeinen Formeln (II), (III), (IV) und (V) R³ und R⁴ für Wasserstoff und den Methylrest stehen.

5. Verfahren nach einem oder mehreren der Ansprüche 1 bis 3 dadurch gekennzeichnet, daß in den allgemeinen Formeln (II), (III), (IV) und (V) R³ und R⁴ gemeinsam und R⁵ und R⁶ gemeinsam für die Gruppierungen (CH)₄, (CH₂)₄, (CH₂)₅ stehen.

6. Verfahren nach einem oder mehreren der Ansprüche 1 bis 5 dadurch gekennzeichnet, daß in den allgemeinen Formeln (IV) und (V) Y für die Methylen-, die Dimethylmethylen-, die Diphenylmethylen-, die 1,3-Phenylen- und die Ethylidengruppe steht.

7. Verfahren nach einem oder mehreren der Ansprüche 1 bis 5 dadurch gekennzeichnet, daß in den allgemeinen Formeln (IV) und (V) Y für die Dimethylsilylen- und die Tetramethyldisilylengruppe steht.

8. Verfahren nach einem oder mehreren der Ansprüche 1 bis 7 dadurch gekennzeichnet, daß in der allgemeinen Formel (I) a 1 oder 2 ist.

9. Verfahren nach einem oder mehreren der Ansprüche 1 bis 8 dadurch gekennzeichnet, daß in der allgemeinen Formel (I) b 1 ist.

10. Verfahren nch einem oder mehreren der Ansprüche 1 bis 9 dadurch gekennzeichnet, daß in der allgemeinen Formel (I) c 0 bis 2 ist.

11. Verfahren nach einem oder mehreren der Ansprüche 1 bis 10 dadurch gekennzeichnet, daß in der allgemeinen Formel (I) n 0 bis 2 ist.

12. Verfahren nach einem oder mehreren der Ansprüche 1 bis 11 dadurch gekennzeichnet, daß in der allgemeinen Formel (I) A für Halogenid- und Pseudohalogenid-Ionen, das Tetraphenylborat-, das Tetrafluoroborat-, das Hexafluorophosphat-, das Acetat-, das Tetracarbonylcobaltat-, das Hexafluoroferrat-, das Tetrachloroferrat-, das Tetrachloroaluminat- und das Tetrachloropalladat-Ion steht.

13. Verfahren nach einem oder mehreren der Ansprüche 1 bis 12 dadurch gekennzeichnet, daß die aromatischen Halogenverbindungen der allgemeinen Formel 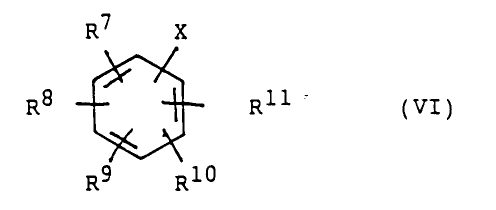 folgen, wobei X Fluor, Chlor, Brom, Jod, R⁷ bis R¹¹ unabhängig voneinander Wasserstoff, Alkylreste mit 1 bis 8 Kohlenstoffatomen, Alkoxyreste mit 1 bis 8 Kohlenstoffatomen, Acyloxyreste mit 1 bis 8 Kohlenstoffatomen, -C₆H₅, OC₆H₅, Fluor, Chlor, Brom, Jod, -OH, -NO₂, -S(O)O₂CF₃, -CN, -COOH, -CHO, -SO₃H, -SO₂ (C₁- bis C₈-alkyl), -SO(C₁- bis C₈-alkyl), -NH₂, -NH(C₁- bis C₈-alkyl), -N(C₁- bis C₈-alkyl)₂, -C(hal)₃ (hal = Halogen), -NHCO(C₁- bis C₄-alkyl), -COO(C₁- bis C₈-alkyl), -CONH₂, -CO(C₁- bis C₈-alkyl), -NHCOOH, -NCOO(C₁- bis C₄-alkyl), -COC₆H₅, -COOC₆H₅, -PO(C₆H₅)₂ und -PO(C₁- bis C₄-alkyl) bedeuten.

14. Verfahren nach Anspruch 13 dadurch gekennzeichnet, daß in der allgemeinen Formel (VI) R⁷ bis R¹¹ unabhängig voneinander Wasserstoff, Alkylreste mit 1 bis 8 Kohlenstoffatomen, Alkoxyreste mit 1 bis 8 Kohlenstoffatomen, -C₆H₅, Fluor, Chlor, -NO₂, -CN, -COOH, -COO(C₁- bis C₈-alkyl), -CONH₂, -CO(C₁- bis C₈-alkyl), -COC₆H₅ und -PO(C₆H₅)₂ sind.

15. Verfahren nach Anspruch 13 dadurch gekennzeichnet, daß in der allgemeinen Formel (VI) einer der Reste R⁷ bis R¹¹ der Gruppierung 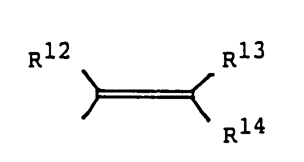 entsprechen, in der R¹² für Wasserstoff, Alkylreste mit 1 bis 8 Kohlenstoffatomen, Alkoxyreste mit 1 bis 8 Kohlenstoffatomen, den Phenylrest oder Fluor steht und R¹³ und R¹⁴ unabhängig voneinander Wasserstoff, -CN, -COOH, -COO(C₁- bis C₈-alkyl), -CONH₂, -CONH(C₁- bis C₄-alkyl), -CON(C₁- bis C₄-alkyl)₂, Fluor, -COOC₆H₅, (C₁- bis C₈-alkyl)C₆H₄, -PO(C₆H₅)₂, -PO[(C₁- bis C₄-alkyl)]₂, -COC₆H₅, -CO(C₁- bis C₄-alkyl), Alkoxyreste mit 1 bis 4 Kohlenstoffatomen, -NH(C₁- bis C₄-alkyl), -PO₃H, -SO₃H, -SO₃(C₁- bis C₄-alkyl), -SO₂(C₁- bis C₄-alkyl) oder -OC₆H₅ sind.

16. Verfahren nach Anspruch 13 dadurch gekennzeichent, daß in der allgemeinen Formel (VI) R⁷, R⁸, R¹⁰ und R¹¹ gleich sind und Wasserstoff bedeuten.

17. Verfahren nach Anspruch 13 dadurch gekennzeichnet, daß in der allgemeinen Formel (VI) R⁹ -CH₃, -OCH₃, -NO₂ oder -C(O)H ist.

18. Verfahren nach einem oder mehreren der Ansprüche 1 bis 17 dadurch gekennzeichnet, daß die Olefine der allgemeinen Formel 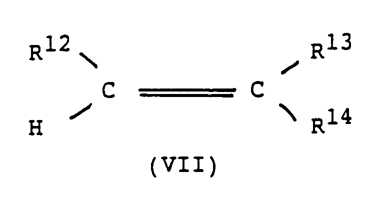 entsprechen, in der R¹² für Wasserstoff, Alkylreste mit 1 bis 8 Kohlenstoffatomen, Alkoxyreste mit 1 bis 8 Kohlenstoffatomen, den Phenylrest oder Fluor steht und R¹³ und R¹⁴ unabhängig voneinander Wasserstoff, -CN, -COOH, -COO(C₁- bis C₈-alkyl), -CONH₂, -CONH(C₁- bis C₄-alkyl), -CON(C₁- bis C₄-alkyl)₂, Fluor, -COOC₆H₅, (C₁- bis C₈-alkyl)C₆H₄, -PO(C₆H₅)₂, -PO[(C₁- bis C₄-alkyl)]₂, -COC₆H₅, -CO(C₁- bis C₄-alkyl), Alkoxyreste mit 1 bis 4 Kohlenstoffatomen, -NH(C₁- bis C₄-alkyl), -PO₃H, -SO₃H, -SO₃(C₁- bis C₄-alkyl), -SO₂(C₁- bis C₄-alkyl) oder -OC₆H₅ sind.

19. Verfahren nach Anspruch 18 dadurch gekennzeichnet, daß in der allgemeinen Formel (VII) R¹² Wasserstoff oder ein Alkylrest mit 1 bis 8 Kohlenstoffatomen ist.

20. Verfahren nach Anspruch 18 dadurch gekennzeichnet, daß in der allgemeinen Formel (VII) R¹³ und R¹⁴ unabhängig voneinander Wasserstoff, -CN, -COOH, -COO(C₁- bis C₈-alkyl), -COOC₆H₅, -COC₆H₅, -CO(C₁- bis C₄-alkyl) sind.

21. Verfahren nach Anspruch 18 dadurch gekennzeichnet, daß in der allgemeinen Formel (VII) R¹² und R¹⁴ gleich sind und Wasserstoff bedeuten.

22. Verfahren nach einem oder mehreren der Ansprüche 1 bis 21 dadurch gekennzeichnet, daß die Umsetzung der Ausgangsstoffe Halogenaromaten und Olefine bei 20 bis 220°C, insbesondere 60 bis 180°C, bevorzugt 100 bis 160°C erfolgt.

23. Verfahren nach einem oder mehreren der Ansprüche 1 bis 22 dadurch gekennzeichnet, daß bei der Umsetzung der Ausgangsstoffe entstehender Halogenwasserstoff durch eine Base gebunden wird.

24. Verfahren nach einem oder mehreren der Ansprüche 1 bis 23 dadurch gekennzeichnet, daß die Umsetzung der Ausgangsstoffe in einem aprotischen Lösungsmittel erfolgt.

25. Verfahren nach einem oder mehreren der Ansprüche 1 bis 24 dadurch gekennzeichnet, daß den Katalysatoren Alkalisalze, Erdalkalisalze, Salze von Übergangsmetallen der 6. bis 8. Nebengruppe des Periodensystems, Tri- oder Tetraalkylammonium, -phosphonium- oder -arsoniumsalze zugesetzt werden.

## Claims

1. A process for preparing monofunctional, difunctional or polyfunctional aromatic olefins by reacting haloaromatics with olefins, which comprises carrying out the reaction at temperatures of from 20 to 220°C in the presence of catalytic compounds which correspond to the formula
[LₐPd_{b}X_{c}]ⁿAₙ (I)
where X are monodentate or multidentate, charged or uncharged ligands bound to palladium as central atom and L, which are likewise bound as ligands to the central atom, are monocarbenes of the formulae 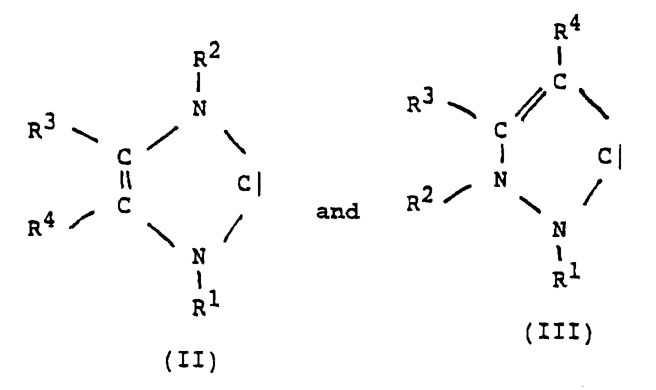 or dicarbenes of the formulae 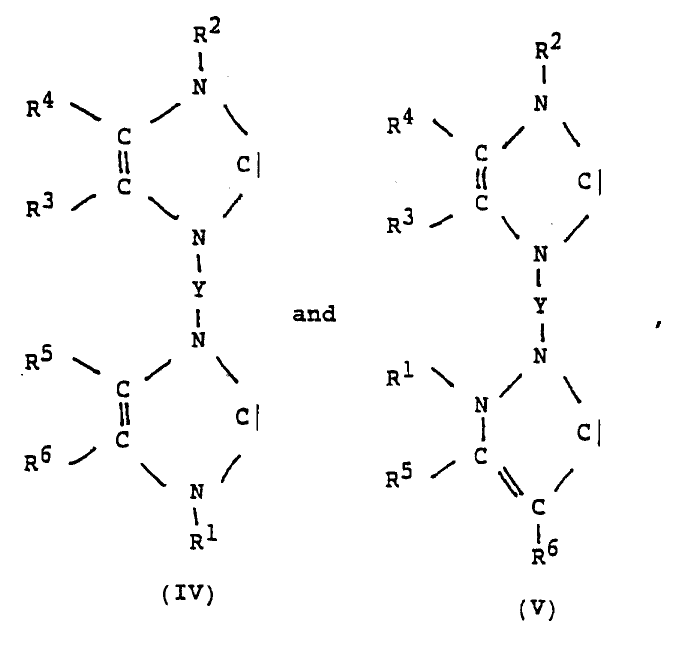 where R¹, R², R³, R⁴, R⁵ and R⁶ are identical or different, straight-chain or branched, sulfonated or unsulfonated alkyl radicals having from 1 to 7 carbon atoms, sulfonated or unsulfonated aliphatic monocyclic or polycyclic radicals having from 5 to 18 carbon atoms, sulfonated or unsulfonated alkenyl radicals having from 2 to 5 carbon atoms, sulfonated or unsulfonated aryl radicals having from 6 to 14 carbon atoms or sulfonated or unsulfonated arylalkyl radicals having from 7 to 19 carbon atoms, R³, R⁴, R⁵ and R⁶ may also be hydrogen, R³ together with R⁴ and R⁵ together with R⁶ may in each case also be identical or different fused and sulfonated or unsulfonated radicals having from 3 to 7 carbon atoms, R¹, R², R⁴ or R⁶ can form a ring with ligands X, Y is a saturated or unsaturated, straight-chain or branched alkylidene radical having from 1 to 4 carbon atoms or a dialkylsilylene or tetraalkyldisilylene radical, A is a singly charged anion or the chemical equivalent of a multiply charged anion, b is an integer from 1 to 3, a is an integer from 1 to 4 · b and c = 0 or an integer from 1 to 4 · b and n = 0 or an integer from 1 to 6.

2. The process as claimed in claim 1, wherein X in the formula (I) is hydrogen, the hydrogen ion, a halogen, a halide ion, a pseudohalide, a carboxylate ion, a sulfonate ion, an alkyl group having from 1 to 7 carbon atoms, an amide radical, an alkoxide radical, an acetylacetonate radical, carbon monoxide, nitrogen monoxide, a nitrile, an isonitrile, a monoolefin or diolefin, an alkyne and a π-aromatic radical.

3. The process as claimed in claim 1 or 2, wherein in the formulae (II), (III), (IV) and (V) R¹, R², R³, R⁴, R⁵, R⁶ are the radicals methyl, isopropyl, tert-butyl, benzyl, triphenylmethyl, phenyl, tolyl, xylyl and mesityl.

4. The process as claimed in one or more of claims 1 to 3, wherein in the formulae (II), (III), (IV) and (V) R³ and R⁴ are hydrogen and the methyl radical.

5. The process as claimed in one or more of claims 1 to 3, wherein in the formulae (II), (III), (IV) and (V) R³ together with R⁴ and R⁵ together with R⁶ are the groups (CH)₄, (CH₂)₄, (CH₂)₅.

6. The process as claimed in one or more of claims 1 to 5, wherein in the formulae (IV) and (V) Y is the methylene, the dimethylmethylene, the diphenylmethylene, the 1,3-phenylene or the ethylidene group.

7. The process as claimed in one or more of claims 1 to 5, wherein in the formulae (IV) and (V) Y is the dimethylsilylene or the tetramethyldisilylene group.

8. The process as claimed in one or more of claims 1 to 7, wherein in the formula (I) a is 1 or 2.

9. The process as claimed in one or more of claims 1 to 8, wherein in the formula (I) b is 1.

10. The process as claimed in one or more of claims 1 to 9, wherein in the formula (I) c is from 0 to 2.

11. The process as claimed in one or more of claims 1 to 10, wherein in the formula (I) n is from 0 to 2.

12. The process as claimed in one or more of claims 1 to 11, wherein in the formula (I) A is a halide or pseudohalide ion, the tetraphenylborate ion, the tetrafluoroborate ion, the hexafluorophosphate ion, the acetate ion, the tetracarbonylcobaltate ion, the hexafluoroferrate ion, the tetrachloroferrate ion, the tetrachloroaluminate ion or the tetrachloropalladate ion.

13. The process as claimed in one or more of claims 1 to 12, wherein the aromatic halogen compounds have the formula 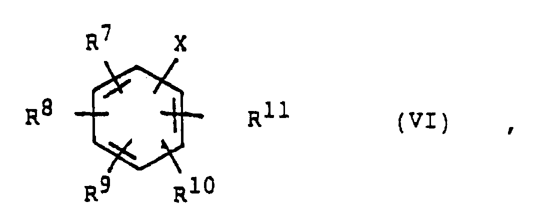 where X is fluorine, chlorine, bromine, iodine, R⁷ to R¹¹ are, independently of one another, hydrogen, alkyl radicals having from 1 to 8 carbon atoms, alkoxy radicals having from 1 to 8 carbon atoms, acyloxy radicals having from 1 to 8 carbon atoms, -C₆H₅, OC₆H₅, fluorine, chlorine, bromine, iodine, -OH, -NO₂, -S(O)O₂CF₃, -CN, -COOH, -CHO, -SO₃H, -SO₂(C₁-C₈-alkyl), -SO(C₁-C₈-alkyl), -NH₂, -NH(C₁-C₈-alkyl), -N(C₁-C₈-alkyl)₂, -C(hal)₃ (hal = halogen), -NHCO(C₁-C₄-alkyl), -COO(C₁-C₈-alkyl), -CONH₂, -CO(C₁-C₈-alkyl), -NHCOOH, -NCOO(C₁-C₄-alkyl), -COC₆H₅, -COOC₆H₅, -PO(C₆H₅)₂ and -PO(C₁-C₄-alkyl).

14. The process as claimed in claim 13, wherein in the formula (VI) R⁷ to R¹¹ are, independently of one another, hydrogen, alkyl radicals having from 1 to 8 carbon atoms, alkoxy radicals having from 1 to 8 carbon atoms, -C₅H₅, fluorine, chlorine, -NO₂, -CN, -COOH, -COO(C₁-C₈-alkyl), -CONH₂, -CO(C₁-C₈-alkyl), -COC₆H₅ and -PO(C₆H₅)₂.

15. The process as claimed in claim 13, wherein in the formula (VI) one of the radicals R⁷ to R¹¹ is the group 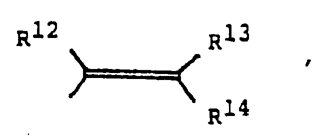 where R¹² is hydrogen, an alkyl radical having from 1 to 8 carbon atoms, an alkoxy radical having from 1 to 8 carbon atoms, the phenyl radical or fluorine and R¹³ and R¹⁴ are, independently of one another, hydrogen, -CN, -COOH, -COO(C₁-C₈-alkyl), -CONH₂, -CONH(C₁-C₄-alkyl), -CON(C₁-C₄-alkyl)₂,_{,} fluorine, -COOC₆H₅, (C₁-C₈-alkyl)C₆H₄, -PO(C₆H₅)₂, -PO[(C₁-C₄-alkyl)]₂, -COC₆H₅, -CO(C₁-C₄-alkyl), alkoxy radicals having from 1 to 4 carbon atoms, -NH(C₁-C₄-alkyl), -PO₃H, -SO₃H, -SO₃(C₁-C₄alkyl), -SO₂(C₁-C₄-alkyl) or -OC₆H₅.

16. The process as claimed in claim 13, wherein in the formula (VI) R⁷, R⁸, R¹⁰ and R¹¹ are identical and are hydrogen.

17. The process as claimed in claim 13, wherein in the formula (VI) R⁹ is -CH₃, -OCH₃, -NO₂ or -C(O)H.

18. The process as claimed in one or more of claims 1 to 17, wherein the olefins have the formula 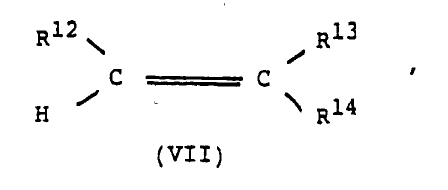 where R¹² is hydrogen, an alkyl radical having from 1 to 8 carbon atoms, an alkoxy radical having from 1 to 8 carbon atoms, the phenyl radical or fluorine and R¹³ and R¹⁴ are, independently of one another, hydrogen, -CN, -COOH, -COO(C₁-C₈-alkyl), -CONH₂, -CONH(C₁-C₄-alkyl), -CON(C₁-C₄-alkyl)₂, fluorine, -COOC₆H₅, (C₁-C₈-alkyl)C₆H₄, -PO(C₆H₅)₂, -PO[(C₁-C₄-alkyl)]₂, -COC₆H₅, -CO(C₁-C₄-alkyl), alkoxy radicals having from 1 to 4 carbon atoms, -NH(C₁-C₄-alkyl), -PO₃H, -SO₃H, -SO₃(C₁-C₄alkyl), -SO₂(C₁-C₄-alkyl) or -OC₆H₅.

19. The process as claimed in claim 18, wherein in the formula (VII) R¹² is hydrogen or an alkyl radical having from 1 to 8 carbon atoms.

20. The process as claimed in claim 18, wherein in the formula (VII) R¹³ and R¹⁴ are, independently of one another, hydrogen, -CN, -COOH, -COO(C₁-C₈-alkyl), -COOC₆H₅, -COC₆H₅, -CO(C₁-C₄-alkyl).

21. The process as claimed in claim 18, wherein in the formula (VII) R¹² and R¹⁴ are identical and are hydrogen.

22. The process as claimed in one or more of claims 1 to 21, wherein the reaction of the starting materials haloaromatics and olefins is carried out at from 20 to 220°C, in particular from 60 to 180°C, preferably from 100 to 160°C.

23. The process as claimed in one or more of claims 1 to 22, wherein hydrogen halide formed in the reaction of the starting materials is bound by a base.

24. The process as claimed in one or more of claims 1 to 23, wherein the reaction of the starting materials is carried out in an aprotic solvent.

25. The process as claimed in one or more of claims 1 to 24, wherein alkali metal salts, alkaline earth metal salts, salts of transition metals of the 6th to 8th transition groups of the Periodic Table, trialkylammonium or tetraalkylammonium, trialkylphosphonium or tetraalkylphosphonium, or trialkylarsonium or tetraalkylarsonium salts are added to the catalysts.

## Revendications

1. Procédé de préparation d'oléfines aromatiques mono-, di- ou polyfonctionnelles par réaction de composés aromatiques halogénés avec des oléfines, caractérisé en ce que l'on effectue la réaction à des températures de 20 à 220°C en présence, comme catalyseurs, de composés correspondant à la formule générale
[LₐPd_{b}X_{c}]ⁿAₙ (I)
dans laquelle X représente des ligands chargés ou non chargés, à une ou plusieurs liaisons de coordination, liés au palladium comme atome central, et
L représente des monocarbènes de formule générale 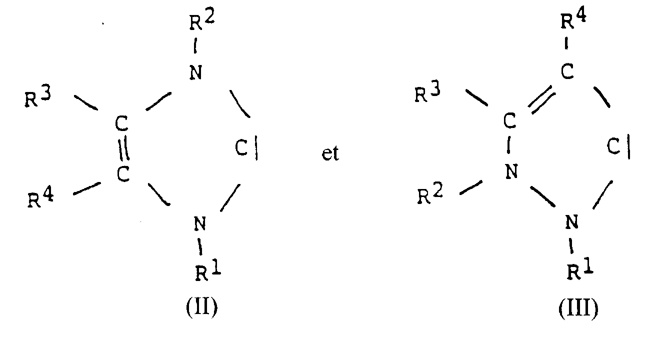 ou des dicarbènes de formule générale 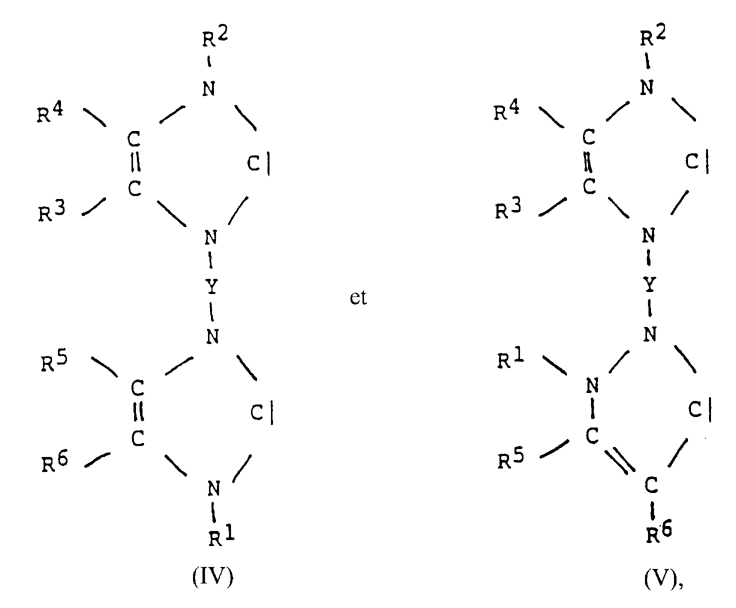 liés aussi en tant que ligands à l'atome central, où R¹, R², R³, R⁴, R⁵ et R⁶ sont identiques ou différents et représentent des restes alkyle linéaires ou ramifiés, éventuellement sulfonés, de 1 à 7 atomes de carbone, des restes mono- ou polycycliques aliphatiques éventuellement sulfonés de 5 à 18 atomes de carbone, des restes alcényle éventuellement sulfonés de 2 à 5 atomes de carbone, des restes aryle éventuellement sulfonés de 6 à 14 atomes de carbone ou des restes arylalkyle éventuellement sulfonés de 7 à 19 atomes de carbone, R³, R⁴, R⁵ et R⁶ représentent aussi des atomes d'hydrogène, R³ et R⁴ ensemble, tout comme R⁵ et R⁶ ensemble, représentent aussi des restes condensés identiques ou différents et éventuellement sulfonés de 3 à 7 atomes de carbone, R¹, R², R⁴ ou R⁶ peuvent former un cycle avec des ligands X,
Y représente un reste alkylidène saturé ou insaturé, linéaire ou ramifié, de 1 à 4 atomes de carbone, ou un reste dialkylsilylène ou tétraalkyldisilylène,
A représente un anion monovalent ou l'équivalent chimique d'un anion polyvalent,
b représente un nombre entier de 1 à 3,
a correspond à un nombre entier de 1 à 4 · b et c = 0 ou correspond à un nombre entier de 1 à 4 · b, et
n = 0 ou est un nombre entier de 1 à 6.

2. Procédé selon la revendication 1, caractérisé en ce que X, dans la formule générale (I), représente l'hydrogène, l'ion hydrogène, des halogènes, des ions halogène, des pseudohalogénures, des ions carboxylate, des ions sulfonate, des groupes alkyle de 1 à 7 atomes de carbone, des restes amide, des restes alkylate, des restes acétylacétonate, le monoxyde de carbone, le monoxyde d'azote, des nitriles, des isonitriles, des mono- ou dioléfines, des alcynes et des restes aromatiques π.

3. Procédé selon la revendication 1 ou 2, caractérisé en ce que, dans les formules générales (II), (III), (IV) et (V), R¹, R², R³, R⁴, R⁵, R⁶ représentent les restes méthyle, isopropyle, tert-butyle, benzyle, triphénylméthyle, phényle, tolyle, xylyle et mésityle.

4. Procédé selon l'une ou plusieurs des revendications 1 à 3, caractérisé en ce que, dans les formules générales (II), (III), (IV) et (V), R³ et R⁴ représentent l'hydrogène et le reste méthyle.

5. Procédé selon l'une ou plusieurs des revendications 1 à 3, caractérisé en ce que, dans les formules générales (II), (III), (IV) et (V), R³ et R⁴ ensemble et R⁵ et R⁶ ensemble représentent les groupements (CH)₄, (CH₂)₄, (CH₂)₅.

6. Procédé selon l'une ou plusieurs des revendications 1 à 5, caractérisé en ce que, dans les formules générales (IV) et (V), Y représente un groupe méthylène, diméthylméthylène, diphénylméthylène, 1,3-phénylène et éthylidène.

7. Procédé selon une ou plusieurs des revendications 1 à 5, caractérisé en ce que, dans les formules générales (IV) et (V), Y représente le groupe diméthylsilylène et le groupe tétraméthyldisilylène.

8. Procédé selon l'une ou plusieurs des revendications 1 à 7, caractérisé en ce que, dans la formule générale (I), a est égal à 1 ou 2.

9. Procédé selon l'une ou plusieurs des revendications 1 à 8, caractérisé en ce que, dans la formule générale (I), b est égal à 1.

10. Procédé selon l'une ou plusieurs des revendications 1 à 9, caractérisé en ce que, dans la formule générale (I), c est un nombre de 0 à 2.

11. Procédé selon l'une ou plusieurs des revendications 1 à 10, caractérisé en ce que, dans la formule générale (I), n représente un nombre de 0 à 2.

12. Procédé selon l'une ou plusieurs des revendications 1 à 11, caractérisé en ce que, dans la formule générale (I), A représente des ions halogénure et pseudohalogénure, l'ion tétraphénylborate, tétrafluoroborate, hexafluorophosphate, acétate, tétracarbonylcobaltate hexafluoroferrate, tétrachloroferrate, tétrachloroaluminate et tétrachloropalladate.

13. Procédé selon l'une ou plusieurs des revendications 1 à 12, caractérisé en ce que les composés aromatiques halogénés correspondent à la formule générale 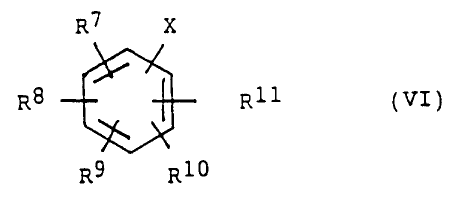 dans laquelle X représente le fluor, le chlore, le brome, l'iode, les R⁷ à R¹¹ sont indépendamment les uns des autres de l'hydrogène, des restes alkyle de 1 à 8 atomes de carbone, des restes alcoxy de 1 à 8 atomes de carbone, des restes acyloxy de 1 à 8 atomes de carbone, -C₆H₅, OC₆H₅, le fluor, le chlore, le brome, l'iode, -OH, -NO₂, -S(O)O₂CF₃, -CN, -COOH, -CHO, -SO₃H, -SO₂(alkyle en C₁-C₈), -SO(alkyle en C₁-C₈), -NH₂, -NH(alkyle en C₁-C₈), -N(alkyle en C₁-C₈)₂, -C(hal)₃ (hal = halogène), -NHCO(alkyle en C₁-C₄), -COO(alkyle en C₁-C₈), -CONH₂, -CO(alkyle en C₁₋C₈), -NHCOOH, -NCOO(alkyle en C₁-C₄), -COC₆H₅, -COOC₆H₅, -PO(C₆H₅)₂ et -PO(alkyle en C₁-C₄).

14. Procédé selon la revendication 13, caractérisé en ce que, dans la formule générale (VI), les R⁷ à R¹¹ sont, indépendamment les uns des autres, de l'hydrogène, des restes alkyle de 1 à 8 atomes de carbone, des restes alcoxy de 1 à 8 atomes de carbone, -C₆H₅, le fluor, le chlore, -NO₂, -CN, -COOH, -COO(alkyle en C₁-C₈), -CONH₂, -CO(alkyle en C₁-C₈), -COC₆H₅, et -PO(C₆H₅)₂.

15. Procédé selon la revendication 13 caractérisé en ce que, dans la formule générale (VI), l'un des restes R⁷ à R¹¹ correspond au groupement 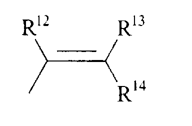 dans lequel R¹² représente l'hydrogène, un reste alkyle de 1 à 8 atomes de carbone, un reste alcoxy de 1 à 8 atomes de carbone, le reste phényle ou le fluor, et R¹³ et R¹⁴ représentent, indépendamment l'un de l'autre, de l'hydrogène, -CN, -COOH, -COO(alkyle en C₁-C₈), -CONH₂, -CONH(alkyle en C₁-C₄), -CON(alkyle en C₁-C₄)₂, du fluor, -COOC₆H₅, (alkyl en C₁-C₈)C₆H₄, -PO(C₆H₅)₂, -PO(alkyle en C₁-C₄)₂, -COC₆H₅, -CO(alkyle en C₁-C₄), des restes alcoxy de 1 à 4 atomes de carbone, -NH(alkyle en C₁-C₄), -PO₃H, -SO₃H, -SO₃(alkyle en C₁-C₄), -SO₂(alkyle en C₁-C₄) ou ―OC₆H₅.

16. Procédé selon la revendication 13, caractérisé en ce que, dans la formule générale (VI), R⁷, R⁸, R¹⁰ et R¹¹ sont identiques et représentent l'hydrogène.

17. Procédé selon la revendication 13, caractérisé en ce que, dans la formule générale (VI), R⁹ est ―CH₃, -OCH₃, -NO, ou ―C(O)H.

18. Procédé selon l'une ou plusieurs des revendications 1 à 17, caractérisé en ce que les oléfines correspondent à la formule générale 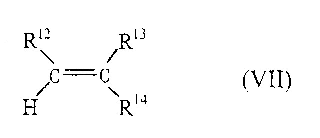 dans laquelle R¹² représente l'hydrogène, un reste alkyle de 1 à 8 atomes de carbone, un reste alcoxy de 1 à 8 atomes de carbone, le reste phényle ou le fluor, et R¹³ et R¹⁴ représentent, indépendamment l'un de l'autre, de l'hydrogène, -CN, -COOH, -COO(alkyle en C₁-C₈), -CONH₂, -CONH(alkyle en C₁-C₄), -CON(alkyle en C₁-C₄)₂, du fluor, -COOC₆H₅, (alkyl en C₁-C₈)C₆H₄, -PO(C₆H₅)₂, -PO(alkyle en C₁-C₄)₂, -COC₆H₅, -CO(alkyle en C₁-C₄), des restes alcoxy de 1 à 4 atomes de carbone, -NH(alkyle en C₁-C₄), -PO₃H, -SO₃H, -SO₃(alkyle en C₁-C₄), -SO₂(alkyle en C₁-C₄) ou ―OC₆H₅.

19. Procédé selon la revendication 18, caractérisé en ce que, dans la formule générale (VII), R¹² représente l'hydrogène ou un reste alkyle de 1 à 8 atomes de carbone.

20. Procédé selon la revendication 18, caractérisé en ce que, dans la formule générale (VII), R¹³ et R¹⁴ sont, indépendamment l'un de l'autre, un atome d'hydrogène, -CN, -COOH, -COO(alkyle en C₁-C₈), -COOC₆H₅, -COC₆H₅, -CO(alkyle en C₁-C₄).

21. Procédé selon la revendication 18, caractérisé en ce que, dans la formule générale (VII), R¹² et R¹⁴ sont identiques et représentent l'hydrogène.

22. Procédé selon l'une ou plusieurs des revendications 1 à 21, caractérisé en ce que la réaction des composés aromatiques halogénés et des oléfines de départ s'effectue à une température de 20 à 220°C, en particulier de 60 à 180°C, de préférence de 100 à 160°C.

23. Procédé selon l'une ou plusieurs des revendications 1 à 22, caractérisé en ce que l'on fixe avec une base l'halogénure d'hydrogène qui se forme lors de la réaction des produits de départ.

24. Procédé selon l'une ou plusieurs des revendications 1 à 23, caractérisé en ce que la réaction des produits de départ s'effectue dans un solvant aprotique.

25. Procédé selon l'une ou plusieurs des revendications 1 à 24, caractérisé en ce que l'on ajoute aux catalyseurs des sels de métaux alcalins, des sels de métaux alcalino-terreux, des sels de métaux de transition du 6^{ème} au 8^{ème} groupe secondaire du système périodique, ou des sels de tri- ou tétraalkylammonium, -phosphonium ou -arsonium.
